(19) Europäisches Patentamt
European Patent Office
Office européen des brevets

(11) Numéro de publication: **0 336 840 B1**

(12) # FASCICULE DE BREVET EUROPEEN

(45) Date de publication de fascicule du brevet: **18.11.93** (51) Int. Cl.[5]: **C07D 305/14**

(21) Numéro de dépôt: **89400934.9**

(22) Date de dépôt: **05.04.89**

(54) **Procédé de préparation du taxol.**

(30) Priorité: **06.04.88 FR 8804512**

(43) Date de publication de la demande:
**11.10.89 Bulletin 89/41**

(45) Mention de la délivrance du brevet:
**18.11.93 Bulletin 93/46**

(84) Etats contractants désignés:
**AT BE CH DE ES FR GB GR IT LI LU NL SE**

(56) Documents cités:
**EP-A- 0 253 738**
**EP-A- 0 253 739**

**JOURNAL OF ORGANIC CHEMISTRY, vol. 51, no. 1, 1986, pages 46-50, American Chemical Society, Washington, US; J.-N. DENIS et al.: "An efficient, enantioselective synthesis of the taxol side chain"**

(73) Titulaire: **CENTRE NATIONAL DE LA RECHERCHE SCIENTIFIOUE (CNRS)**
**15, Ouai Anatole France**
**F-75700 Paris Cedex 07(FR)**

(72) Inventeur: **Denis, Jean-Noel**
**Gites de Belledonne, Apt. no. 3**
**Le Pinet**
**F-38410 Uriage(FR)**
Inventeur: **Greene, Andrew Elliot**
**La Maison du Verger**
**St. Martin d'Uriage**
**F-38410 Uriage(FR)**
Inventeur: **Guenard, Daniel**
**19 rue d'Arcueil**
**F-92120 Montrouge(FR)**
Inventeur: **Gueritte-Voegelein, Françoise**
**19 Avenue de la Frileuse**
**Gometz le Chatel**
**F-91940 Les Ulis(FR)**

(74) Mandataire: **Pilard, Jacques et al**
**RHONE-POULENC SANTE,**
**Service Brevets,**
**20 Avenue Raymond Aron**
**F-92165 Antony Cédex (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 336 840 B1

## Description

La présente invention concerne un procédé de préparation du taxol par hémisynthèse à partir de la désacétyl-10 baccatine III et de la baccatine.

Parmi les dérivés du taxane qui répondent à la formule générale :

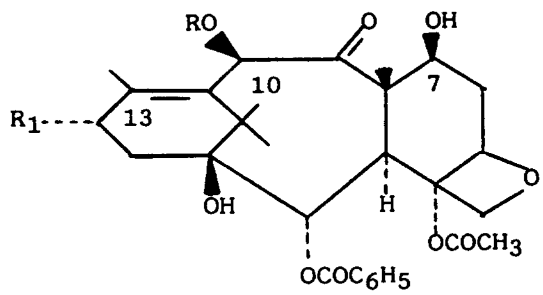

(I)

le taxol est celui pour lequel R représente un radical acétyle et $R_1$ représente un radical -OCO-CHOH-CH-($C_6H_5$)-NHCO$C_6H_5$ (2'R,3'S), la désacétyl-10 baccatine III est celui pour lequel R représente un atome d'hydrogène et $R_1$ représente un radical hydroxy et la baccatine III est celui pour lequel R représente un radical acétyle et $R_1$ représente un radical hydroxy.

Alors que le taxol présente in vitro des propriétés remarquables comme promoteur de la polymérisation de la tubuline et comme inhibiteur de la dépolymérisation des tubules et, de ce fait, constitue un agent antileucémique et antitumoral particulièrement intéressant, la désacétyl-10 baccatine III et la baccatine III ne manifestent pas ces activités.

Le taxol et la baccatine III sont extraits difficilement et avec des rendements généralement faibles, de l'ordre de 100 mg/kg dans le cas du taxol, des écorces de tronc de différentes espèces de Taxus.

La baccatine III se trouve en quantité plus importante dans le bois de ces différentes espèces végétales.

En revanche, la désacétyl-10 baccatine III est extraite beaucoup plus facilement et avec de meilleurs rendements (300 mg/kg de feuilles) à partir des feuilles d'ifs.

Il est donc particulièrement intéressant de pouvoir disposer d'un procédé permettant de préparer le taxol à partir de la désacétyl-10 baccatine III qui est facilement accessible et dont l'obtention ne nécessite pas la destruction totale de l'espèce végétale.

Dans la demande de brevet européen EP 253 739 a été décrite la préparation du taxol et du désacétyl-10 taxol à partir d'un dérivé du taxane de formule générale :

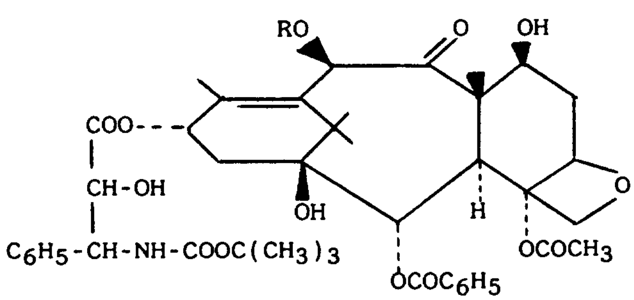

(II)

dont la préparation, à partir de la baccatine III ou de la désacétyl-10 baccatine III, qui fait l'objet de la demande de brevet européen EP 253 738, nécessite la séparation intermédiaire des diastéréoisomères. Il en résulte que la totalité de la baccatine III ou de la désacétyl-10 baccatine III mise en oeuvre ne peut pas conduire au taxol ayant la configuration convenable.

L'article publié dans J. Org. Chem., 51(1)46-50 (1986) décrit la préparation de la chaîne latérale du taxol de formule :

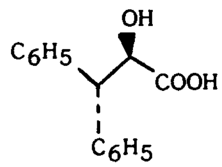

(IIa)

éventuellement sous forme d'ester et suggère seulement son utilisation pour effectuer une synthèse partielle du taxol à partir de la désacétyl-10 baccatine III sans pour autant donner des conditions permettant de réaliser cette synthèse partielle.

Il a maintenant été trouvé, et c'est ce qui fait l'objet de la présente invention, que le taxol peut être obtenu avec de bons rendements par estérification d'un dérivé de la phényl-3 isosérine (2R,3S) de formule générale :

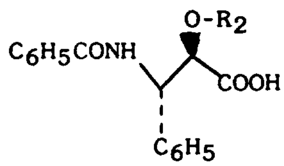

(III)

dans laquelle $R_2$ représente un groupement protecteur de la fonction hydroxy choisi parmi les radicaux méthoxyméthyle, éthoxy-1 éthyle, benzyloxyméthyle, ($\beta$-triméthylsilyléthoxy) méthyle, tétrahydropyrannyle et trichloro-2,2,2 éthoxycarbonyle, sur un dérivé du taxane de formule générale :

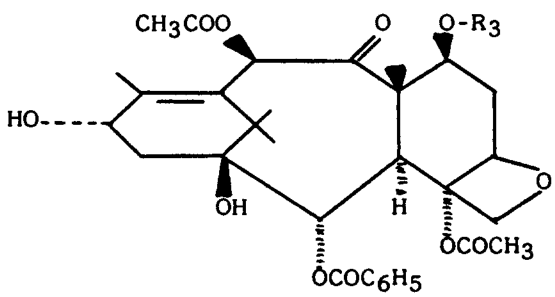

(IV)

dans laquelle $R_3$ représente un groupement protecteur de la fonction hydroxy choisi parmi les radicaux trialkylsilyl dont chaque partie alkyle contient 1 à 3 atomes de carbone, pour obtenir l'ester de formule générale :

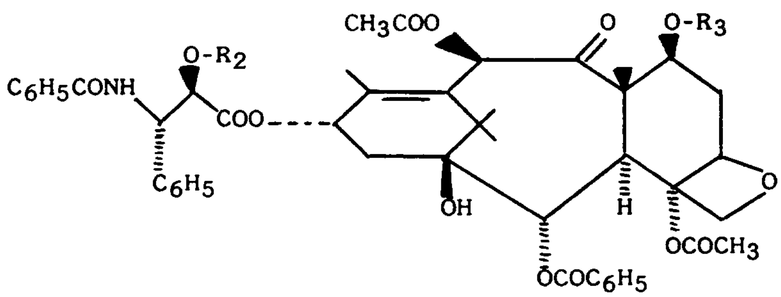

(V)

dans laquelle $R_2$ et $R_3$ sont définis comme précédemment, suivie du remplacement des groupements protecteurs $R_2$ et $R_3$ par un atome d'hydrogène.

Dans la formule générale (III), le radical $R_2$ est, de préférence, le radical éthoxy-1 éthyle.

Dans la formule générale (IV), le radical $R_3$ représente, de préférence, le radical triméthylsilyle ou triéthylsilyle.

Généralement, l'estérification du dérivé du taxane de formule générale (IV) par l'acide de formule générale (III) est effectuée en présence d'un agent de condensation tel qu'un carbodiimide comme le dicyclohexylcarbodiimide ou un carbonate réactif comme le dipyridyl-2 carbonate et d'un agent d'activation tel qu'une dialcoylaminopyridine comme la diméthylamino-4 pyridine en opérant dans un solvant aromatique tel que le benzène, le toluène, les xylènes, l'éthylbenzène, l'isopropylbenzène ou le chlorobenzène à une température comprise entre 60 et 90 ° C.

Généralement, on utilise un excès d'acide de formule générale (III) et d'agent de condensation (dicyclohexylcarbodiimide, dipyridyl-2 carbonate), de préférence 6 à 10 moles par mole de dérivé du taxane de formule générale (IV) et su moins une mole, de préférence 2 à 4 moles, de diméthylamino-4 pyridine par mole de dérivé du taxane de formule générale (IV).

L'élimination des groupements protecteurs de l'ester (2'R, 3'S) obtenu de formule générale :

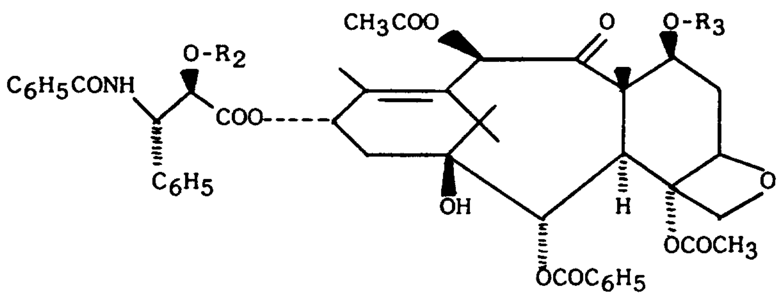

(V)

dans laquelle $R_2$ et $R_3$ sont définis comme précédemment, est généralement effectuée par traitement en milieu acide. Il est particulièrement avantageux d'utiliser un acide tel que l'acide chlorhydrique en solution dans un alcool aliphatique contenant 1 à 3 atomes de carbone (méthanol, éthanol, propanol, isopropanol) à une température voisine de 0 ° C.

Le produit de formule générale (III) peut être obtenu par saponification d'un ester (2R, 3S) de formule générale :

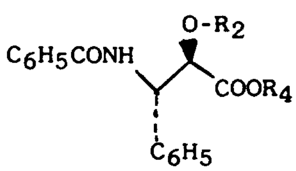

(VI)

dans laquelle $R_2$ est défini comme précédemment et $R_4$ représente un radical alcoyle contenant 1 à 4 atomes de carbone et, de préférence, un radical méthyle, au moyen d'une base minérale telle qu'un hydroxyde de métal alcalin (lithine, soude), un carbonate ou bicarbonate de métal alcalin (bicarbonate de sodium, carbonate de potassium) en milieu hydro-alcoolique tel qu'un mélange éthanol-eau ou méthanoleau, en opérant à une température comprise entre 10 et 40 ° C et de préférence voisine de 25 ° C.

Le produit de formule générale (VI) peut être obtenu dans les conditions habituelles de préparation des éthers et, plus particulièrement selon les procédés décrits par J.N. Denis et coll., J. Org. Chem., 51, 46-50 (1986).

Le produit de formule générale (IV) peut être obtenu par action d'un halogénotrialkylsilane sur la baccatine III ou sur la désacétyl-10 baccatine III suivie, dans ce dernier cas, de l'acétylation de la trialkylsilyl-7 désacétyl-10 baccatine III intermédiairement obtenue.

Généralement, la réaction de l'halogénotrialkylsilane sur la baccatine III ou sur la désacétyl-10 baccatine III s'effectue à une température voisine de 20 ° C en opérant dans un solvant organique basique tel que la pyridine ou dans un solvant organique inerte tel que le chloroforme ou le dichloroéthane en présence d'une amine tertiaire telle que la triéthylamine, la base de Hunig ou la pyridine.

L'acétylation de la trialkylsilyl-7 désacétyl-10 baccatine III est généralement effectuée au moyen de chlorure d'acétyle en opérant à une température voisine de 0 ° C dans un solvant organique basique tel que la pyridine ou dans un solvant organique inerte tel que le chlorure de méthylène, le chloroforme ou le

dichloroéthane en présence d'une amine tertiaire telle que la pyridine ou la base de Hunig.

L'exemple suivant, donné à titre non limitatif, montre comment l'invention peut être mise en pratique.

EXEMPLE

Dans un ballon de 5 cm3 muni d'une agitation magnétique, on introduit, sous atmosphère d'argon, 42,8 mg (0,12 mmole) de N-benzoyl O-(éthoxy-1 éthyl) phényl-3 isosérine dans 1 cm3 de toluène anhydre. On ajoute ensuite 25,9 mg (0,12 mmole) de dipyridyl-2 carbonate. On laisse réagir pendant 4 à 5 minutes puis on ajoute, en une seule fois, 4,9 mg (0,04 mmole) de diméthylamino-4 pyridine et 14 mg (0,02 mmole) de triéthylsilyl-7 baccatine III. On laisse la solution homogène et incolore pendant 3 à 4 minutes puis la chauffe pendant 10 heures à 72-74 °C. Après refroidissement, on dilue le mélange réactionnel par addition d'acétate d'éthyle. On lave la solution organique 3 fois avec une solution aqueuse saturée de bicarbonate de sodium, 2 fois à l'eau puis 2 fois avec une solution saturée de chlorure de sodium. La phase organique est séchée sur sulfate de sodium anhydre. Après filtration et élimination des solvants sous pression réduite (20 mm de mercure ; 2,7 kPa), le résidu obtenu est purifié par chromatographie analytique sur couche mince de silice en éluant avec un mélange etherchlorure de méthylène (5-95 en volumes) en effectuant 4 passages. On obtient ainsi, avec un rendement de 40 %, 8,4 mg (0,0081 mmole) d'ester de formule générale (V) dans laquelle $R_2$ représente un radical éthoxy-1 éthyle et $R_3$ représente un radical triéthylsilyle, sous forme d'un mélange de 2 épimères dans le rapport 60/40, fondant à 169-173 °C après recristallisation dans un mélange chlorure de méthylène-pentane.

On récupère 5,8 mg de triéthylsilyl-7 baccatine III. L'ester obtenu présente les caractéristiques suivantes :

- pouvoir rotatoire : $[\alpha]_D^{24}$ = -33,7 ° (c = 0,41 ; méthanol)
- spectre infra-rouge (film) : 3450, 3300, 3060, 3025, 2950, 2930, 2900, 2870, 1740, 1720, 1640, 1600, 1580, 1520, 1480, 1450, 1365, 1310, 1260, 1240, 1175, 1140, 1105, 1090, 1080, 1020, 980, 945, 820 et 710 $cm^{-1}$
- spectre de résonance magnétique nucléaire du proton (300 MHz ; chloroforme deutéré ; déplacement chimique en ppm ; constantes de couplage J en Hz) :
  0,53-0,62 (m, 6H) ; 0,93 (t, J = 8, 9H) ; 1,00 (t, J = 7, épimère minoritaire 3H) ; 1,04 (t, J = 7, épimère majoritaire 3H) ; 1,18 (épimère majoritaire) et 1,19 (épimère minoritaire) (2s, 3H) ; 1,22 (s, 3H) ; 1,20 et 1,29 (2d, J = 5,3, 3H) ; 1,70 (s, 3H) ; 1,85-1,95 (m, 1H) ; 2,00 et 2,01 (2d, J = 1,2, 3H) ; 2,05-2,20 (m, 1H) ; 2,16 (s, 3H) ; 2,26-2,40 (m, 1H) ; 2,40 (épimère majoritaire) et 2,53 (épimère minoritaire) (2s, 3H) ; 2,46-2,59 (m, 1H) ; 3,04-3,44 (m, 2H) ; 3,81 (épimère majoritaire) et 3,83 (épimère minoritaire) (2d, J = 7, 1H) ; 4,24 (épimère minoritaire) et 4,26 (épimère majoritaire) (2ABq, $J_{AB}$ = 8,1 $\delta_A$-$\delta_B$ = 34, 2H) ; 4,47 (dd, J = 6,6 et 10,6, 1H) ; 4,64 (épimère minoritaire) et 4,72 (épimère majoritaire) (2d, J = 2,7 et 3,7, 1H) ; 4,54 (épimère minoritaire) et 4,80 (épimère majoritaire) (2q, J = 5,3, 1H) ; 4,94 (ps.t, J = 6,7 et 7,3, 1H) ; 5,68-5,76 (m, 2H) ; 6,24 (ps.t, J = 8 et 9, 1H) ; 6,44 (s, 1H) ; 7,08 (épimère minoritaire) et 7,18 (épimère majoritaire) (2d, J = 8,6 et 8,1, 1H) ; 7,28-7,53 (m, 10H) ; 7,57-7,63 (m, 1H) ; 7,77-7,80 (m, 2H) ; 8,10-8,15 (m, 2H)
- spectre de masse (FAB ; matrice NBA) : m/e = 1040 ($MH^+$)
-

| analyse élémentaire : $C_{57}H_{73}O_{15}SiN$ | | | | | | |
|---|---|---|---|---|---|---|
| Calculé %<br>Trouvé | C | 65,81<br>65,57 | H | 7,07<br>7,34 | N | 1,35<br>1,62 |

Dans un ballon de 10 cm3 muni d'une agitation magnétique, on introduit, à 0 °C sous atmosphère d'argon, 72 mg (0,009 mmole) de l'ester obtenu précédemment. On ajoute 3,6 cm3 d'une solution d'acide chlorhydrique dans l'éthanol à 0,5 % préalablement refroidie à 0 °C. On agite à 0 °C pendant 30 heures. La réaction terminée, on dilue le mélange réactionnel par addition d'acétate d'éthyle à 0 °C puis on ajoute de l'eau. Après décantation, la phase organique est lavée 5 fois à l'eau et 2 fois avec une solution saturée de chlorure de sodium puis elle est séchée sur sulfate de sodium anhydre. Après filtration, les solvants sont éliminés sous pression réduite (20 mm de mercure ; 2,6 kPa). Le résidu obtenu (72 mg) est purifié par chromatographie préparative sur couche mince de silice en éluant avec un mélange dichlorométhane-méthanol (90-10 en volumes). On obtient ainsi 54 mg (0,063 mmole) de taxol.

Le rendement est de 91 %.

Le taxol ainsi obtenu présente les caractéristiques suivantes :

- pouvoir rotatoire : $[\alpha]_D^{24}$ = -49,7° (c = 0,36 ; méthanol)
- spectre infra-rouge (film) : 3400, 3060, 3025, 3000, 2950, 2900, 1740, 1720, 1650, 1600, 1580, 1520, 1480, 1450, 1370, 1315, 1260, 1240, 1180, 1110, 1070, 1030, 980, 950, 905, 800 et 710 $cm^{-1}$
- spectre de résonance magnétique nucléaire du proton (300 MHz ; chloroforme deutéré ; déplacements chimiques en ppm ; constantes de couplage en Hz) :
  1,15 (s, 3H) ; 1,24 (s, 3H) ; 1,69 (s, 3H) ; 1,80 (s, 3H) ; 1,83 (s, 1H) ; 1,83-1,93 (m, 1H) ; 2,24 (s, 3H) ; 2,28-2,39 (m, 2H) ; 2,39 (s, 3H) ; 2,47 (d, J = 4, 1H) ; 2,48-2,57 (m, 1H) ; 3,55 (d, J = 5, 1H) ; 3,80 (d, J = 7, 1H) ; 4,25 (ABq, $J_{AB}$ = 8,4, $\delta_A$-$\delta_B$ = 32, 2H) ; 4,37-4,44 (m, 1H) ; 4,80 (dd, J = 2,5 et 5, 1H) ; 4,95 (d, J = 7,7, 1H) ; 5,68 (d, J = 7, 1H) ; 5,79 (dd, J = 2,5 et 8,8, 1H) ; 6,23 (t, J = 9, 1H) ; 6,27 (s, 1H) ; 6,98 (d, J = 8,8, 1H) ; 7,33-7,54 (m, 10H) ; 7,59-7,64 (m, 1H) ; 7,72-7,75 (m, 2H) ; 8,12-8,15 (m, 2H)
- spectre de résonance magnétique nucléaire du $^{13}C$ (chloroforme deutéré) :
  9,57 ($CH_3$) ; 14,83 ($CH_3$) ; 20,83 ($CH_3$) ; 21,62 ($CH_3$) ; 22,85 ($CH_3$) ; 26,69 ($CH_3$) ; 35,65 ($CH_2$) ; 35,73 ($CH_2$) ; 43,20 (C) ; 45,86 (CH) ; 55,05 (CH) ; 58,67 (C) ; 72,20 (CH) ; 72,41 (CH) ; 73,23 (CH) ; 75,00 (CH) ; 75,58 (CH) ; 76,58 ($CH_2$) ; 79,10 (C) ; 81,21 (C) ; 84,42 (CH) ; 127,06 (CH) ; 128,38 (CH) ; 128,72 (CH) ; 129,04 (CH) ; 129,21 (C) ; 130,22 (CH) ; 131,97 (CH) ; 133,26 (C) ; 133,71 (CH) ; 138,03 (C) ; 141,98 (C) ; 167,04 (C) ; 170,37 (C) ; 171,22 (C) ; 172,73 (C) ; 203,62 (C)
- spectre de masse (FAB ; matrice NAB) : m/e = 854 ($MH^+$).

La N-benzoyl O-(éthoxy-1 éthyl) phényl-3 isosérine (2R, 3S) peut être obtenue de la manière suivante :

Dans un ballon de 100 cm3 muni d'une agitation magnétique contenant 30 cm3 de méthanol, on ajoute 380 mg d'ester méthylique de la N-benzoyl O-(éthoxy-1 éthyl) phényl-3 isosérine. A la solution obtenue, on ajoute 15 cm3 d'eau distillée et 414 mg (3 mmoles) de carbonate de potassium solide. On agite pendant 40 heures à 25°C puis on évapore le méthanol sous pression réduite. La phase aqueuse résiduelle est extraite plusieurs fois à l'éther. La phase aqueuse est acidifiée par une solution aqueuse d'acide chlorhydrique à 10 % (p/v) puis elle est extraite au dichlorométhane. Les phases organiques réunies sont lavées plusieurs fois à l'eau puis avec une solution saturée de chlorure de sodium. Les phases organiques sont séchées sur sulfate de magnésium anhydre. Après filtration et élimination du solvant sous pression réduite, on obtient 254 mg (0,711 mmole) de N-benzoyl O-(éthoxy-1 éthyl) phényl-3 isosérine dont les caractéristiques sont les suivantes :

- point de fusion : 93-94°C
- spectre infra-rouge (film) : 3425, 3600-2100, 3060, 3025, 2950, 2925, 1740, 1640, 1600, 1580, 1520, 1480, 1440, 1300, 1140, 1075, 1020, 950, 920, 865, 800, 770 et 700 $cm^{-1}$
- spectre de résonance magnétique nucléaire du proton (300 MHz ; chloroforme deutéré ; déplacements chimiques en ppm ; constantes de couplage J en Hz) :
  0,90 et 1,07 (2t, J = 7, 3H) ; 1,24 (d, J = 5,3, 3H) ; 2,88-2,99 et 3,24-3,45 (2m, 2H) ; 4,50 et 4,63 (2d, J = 2,4, 1H) ; 4,60 et 4,81 (2q, J = 5,3, 1H) ; 5,74-5,80 (m, 1H) ; 7,26-7,52 (m,4H) ; 7,48-7,83 (m, 2H) ; 7,0-7,8 (s large, 1H).

L'ester méthylique de la N-benzoyl O-(éthoxy-1 éthyl) phényl-3 isosérine (2R, 3S) peut être préparé de la manière suivante :

Dans un ballon de 25 cm3 muni d'une agitation magnétique, on introduit successivement 299 mg (1 mmole) d'ester méthylique de la N-benzoyl phényl-3 isosérine, 10 cm3 de dichlorométhane sec, 25,1 mg (0,1 mmole) de p.toluènesulfonate de pyridinium et 956,4 µl (721 mg, 10 mmoles) d'éthylvinyléther. On agite le mélange réactionnel pendant 3 heures à 25°C. La réaction terminée, on ajoute 1 goutte de pyridine puis on dilue le mélange réactionnel par addition de dichlorométhane. On lave la phase organique 2 fois à l'eau puis avec une solution saturée de chlorure de sodium et la sèche sur sulfate de sodium anhydre. Après filtration et élimination des solvants sous pression réduite, on obtient 380 mg d'ester méthylique de la N-benzoyl O-(éthoxy-1 éthyl) phényl-3 isosérine sous forme d'un mélange équimoléculaire de 2 épimères dont les caractéristiques sont les suivantes :

- point de fusion : 124-125°C (après recristallisation dans un mélange dichlorométhane-pentane)
- pouvoir rotatoire : $[\alpha]_D^{23}$ = -25,9° (c = 0,54 ; méthanol)
- spectre infra-rouge (film) : 3350, 3060, 3025, 2980, 2940, 1740, 1635, 1600, 1580, 1530, 1490, 1435, 1380, 1340, 1320, 1275, 1242, 1198, 1175, 1150, 1080, 1030, 990, 955, 900, 800, 702 et 698 $cm^{-1}$
- spectre de résonance magnétique nucléaire du proton (300 MHz ; chloroforme deutéré ; déplacements chimiques en ppm ; constantes de couplage J en Hz) :
  0,87 et 0,98 (2t, J = 7, 3H) ; 1,13 et 1,22 (2d, J = 5,4, 3H) ; 2,82 et 2,89 et 3,22-3,36 (m, 2H) ; 3,755 et 3,76 (2s, 3H) ; 4,48 et 4,60 (2d, J = 2,4, 1H) ; 4,50 et 4,78 (2q, J = 5,4, 1H) ; 5,64 et 5,68 (2dd, J = 2,4 et 8,2, 1H) ; 7,18 et 7,19 (2d, J = 8,2, 1H) ; 7,23-7,55 (m, 8H) ; 7,80-7,84 (m, 2H)
- spectre de masse (FAB ; matrice NBA) : m/e = 372 ($MH^+$)

-

| analyse élémentaire : $C_{21}H_{25}O_5N$ | | | | | | |
|---|---|---|---|---|---|---|
| Calculé % | C | 67,90 | H | 6,78 | N | 3,77 |
| Trouvé | | 67,98 | | 6,94 | | 3,75 |

L'ester méthylique de la N-benzoyl phényl-3 isosérine peut être préparé selon J.N. Denis et coll., J. Org. Chem., 51, 46-50 (1986).

La triéthylsilyl-7 baccatine III peut être préparée selon l'une des manières suivantes :

a) Dans un ballon de 100 cm3 muni d'une agitation magnétique, on introduit, sous atmosphère d'argon, 544 mg (1 mmole) de désacétyl-10 baccatine III en solution dans 50 cm3 de pyridine anhydre. On ajoute ensuite 3,36 cm3 (3,014 g, 20 mmoles) de chlorure de triéthylsilyle. On agite le mélange réactionnel homogène et jaune pendant 24 heures à 0 °C. On ajoute alors de l'acétate d'éthyle et de l'eau. Après décantation, la phase aqueuse est extraite avec de l'acétate d'éthyle. Les phases organiques réunies sont traitées par une solution aqueuse saturée de sulfate de cuivre jusqu'à élimination totale de la pyridine. Les phases organiques sont lavées à l'eau puis avec une solution saturée de chlorure de sodium puis séchées sur sulfate de sodium anhydre. Après filtration et évaporation des solvants sous pression réduite, on obtient 2,73 g d'un produit qui est purifié sur une colonne de silice en éluant avec un mélange dichlorométhane-méthanol (99-1 en volumes). On obtient ainsi, avec un rendement de 78 %, 512 mg (0,778 mmole) de désacétyl-10 triéthylsilyl-7 baccatine III sous forme d'un solide blanc dont les caractéristiques sont les suivantes :

- point de fusion : 256-257 °C (après recristallisation dans un mélange dichlorométhane-pentane)
- pouvoir rotatoire : $[\alpha]_D^{23}$ = -23,6 ° (c = 0,41 méthanol)
- spectre infra-rouge (film) : 3450, 2950, 2875, 1735, 1705, 1600, 1580, 1450, 1380, 1275, 1242, 1180, 1140, 1115, 1100, 1075, 1060, 1030, 1020, 1000, 990, 950, 925, 885, 860, 822, 740 et 715 $cm^{-1}$
- spectre de résonance magnétique nucléaire du proton (300 MHz ; chloroforme deutéré ; déplacements chimiques en ppm ; constantes de couplage J en Hz) :
  0,48-0,70 (m, 6H) ; 0,94 (t, J = 8, 9H) ; 1,08 (s, 6H) ; 1,57 (s, 1H) ; 1,74 (s, 3H) ; 1,86-1,95 (m, 1H) ; 2,02 (d, J = 5, 1H) ; 2,09 (d, J = 1,1, 3H) ; 2,25-2,35 (m, 2H) ; 2,28 (s, 3H) ; 2,42-2,52 (m, 1H) ; 3,95 (d, J = 7, 1H) ; 4,24 (ABq, $J_{AB}$ = 8,2, $\delta_A$-$\delta_B$ = 42, 2H) ; 4,24 (d,J = 2, 1H) ; 4,41 (dd,J = 6,6 et 10,6, 1H) ; 4,85-4,90 (m, 1H) ; 4,95 (dd, J = 1,9 et 9,6, 1H) ; 5,17 (d, J = 2, 1H) ; 5,60 (d, J = 7, 1H) ; 7,44-7,50 (m, 2H) ; 7,57-7,62 (m, 1H) ; 8,08-8,11 (m, 2H)
- spectre de résonance magnétique nucléaire du $^{13}C$ (chloroforme deutéré) :
  5,16 ($CH_2$) ; 6,72 ($CH_3$) ; 9,92 ($CH_3$) ; 15,13 ($CH_3$) ; 19,50 ($CH_3$); 22,60 ($CH_3$) ; 26,86 ($CH_3$) ; 37,26 ($CH_2$) ; 38,64 ($CH_2$) ; 42,70 (C) ; 46,99 (CH) ; 57,96 (C) ; 67,95 (CH) ; 72,95 (CH) ; 74,68 (CH) ; 74,83 (CH) ; 76,57 ($CH_2$) ; 78,78 (C) ; 80,75 (C) ; 84,25 (CH) ; 128,57 (CH) ; 129,44 (C) ; 130,07 (CH) ; 133,57 (CH) ; 135,20 (C) ; 141,78 (C) ; 167,04 (C) ; 170,76 (C) ; 210,31 (C)
- spectre de masse (FAB ; matrice NBA) : m/e = 659 ($MH^+$)
-

| analyse élémentaire : $C_{35}H_{50}O_{10}Si$ | | | | | | |
|---|---|---|---|---|---|---|
| Calculé %<br>Trouvé | C | 63,80<br>63,57 | H | 7,65<br>7,72 | Si | 4,26<br>4,04 |

Dans un ballon de 10 cm3 muni d'une agitation magnétique, on introduit, sous atmosphère d'argon, 43,9 mg (0,075 mmole) de désacétyl-10 triéthylsilyl-7 baccatine III en solution dans 1,87 cm3 de pyridine anhydre. On refroidit à 0 ° C, puis on ajoute goutte à goutte, 26,6 µl (29,4 mg ; 0,375 mmole) de chlorure d'acétyle. On agite le mélange qui devient hétérogène pendant 20 heures à 0 ° C. On ajoute à nouveau 26,6 µl de chlorure d'acétyle puis agite pendant 20 heures à 0 ° C. On ajoute de l'acétate d'éthyle puis de l'eau à 0 ° C. Après décantation, la phase aqueuse est extraite 2 fois avec de l'acétate d'éthyle. Les phases organiques réunies sont lavées avec une solution aqueuse saturée de sulfate de cuivre jusqu'à élimination totale de la pyridine puis à l'eau puis avec une solution saturée de chlorure de sodium et enfin séchées sur sulfate de sodium anhydre. Après filtration et élimination des solvants sous pression réduite, le résidu obtenu (65 mg) est purifié par chromatographie sur une colonne de gel de silice en éluant avec un mélange dichlorométhane-méthanol (99-1 en volumes). On obtient ainsi, avec un rendement de 86 %, 45 mg (0,0643 mmole) de triéthylsilyl-7 baccatine III dont les caractéristiques sont les suivantes :

- point de fusion : 253-254 ° C (après recristallisation dans un mélange dichlorométhane-pentane)
- pouvoir rotatoire : $[\alpha]_D^{23}$ = -48,6 ° (c = 0,36 ; méthanol)
- spectre infra-rouge (film) : 3500, 2950, 2875, 1720, 1600, 1580, 1450, 1370, 1270, 1240, 1180, 1140, 1110, 1100, 1075, 1050, 1020, 990, 970, 950, 820, 740 et 710 $cm^{-1}$
- spectre de résonance magnétique nucléaire du proton (300 MHz ; chloroforme deutéré ; déplacements chimiques en ppm ; constantes de couplage J en Hz) :
  0,52-0,65 (m, 6H) ; 0,93 ((t, J = 8, 9H) ; 1,05 (s, 3H) ; 1,20 (s, 3H) ; 1,61 (s, 1H) ; 1,68 (s, 3H) ; 1,83-1,92 (m, 1H) ; 2,01 (d, J = 5, 1H) ; 2,17 (s, 3H) ; 2,19 (d, J = 1,1, 3H) ; 2,24-2,28 (m, 2H) ; 2,28 (s, 3H) ; 2,41-2,58 (m, 1H) ; 3,88 (d, J = 7, 1H) ; 4,23 (ABq, $J_{AB}$ = 8,1, $\delta_A$-$\delta_B$ = 45, 2H) ; 4,45 (dd, J = 6,6 et 10,5, 1H) ; 4,83 (m, 1H) ; 4,96 (d, J = 9,6, 1H) ; 5,63 (d, J = 7, 1H) ; 6,46 (s, 1H) ; 7,45- 7,50 (m, 2H) ; 7,57-7,63 (m, 1H) ; 8,09-8,12 (m, 2H)
- spectre de résonance magnétique nucléaire du $^{13}C$ (chloroforme deutéré) : 5,28 ; 6,72 ; 9,93 ; 14,93 ; 20,06 ; 20,93 ; 22,68 ; 26,83 ; 37,24 ; 38,24 ; 42,79 ; 47,24 ; 58,67 ; 68,00 ; 72,35 ; 74,35 ; 75,77 ; 76,57 ; 78,73 ; 80,89 ; 84,22 ; 128,57 ; 129,38 ; 130,09 ; 132,78 ; 133,61 ; 143,83 ; 167,12 ; 169,33 ; 170,76 et 202,12
- spectre de masse (FAB ; matrice NBA) : m/e = 701 ($MH^+$)
-

| analyse élémentaire : $C_{37}H_{52}O_{11}Si$ | | | | | | |
|---|---|---|---|---|---|---|
| Calculé %<br>Trouvé | C | 63,40<br>63,50 | N | 7,48<br>7,59 | Si | 4,01<br>3,94 |

b) Dans un ballon de 25 cm3 muni d'une agitation magnétique, on introduit, sous atmosphère d'argon, 250 mg (0,4266 mmole) de baccatine III en solution dans 8,5 cm3 de pyridine anhydre. On ajoute ensuite 1,43 cm3 (1,286 g ; 8,53 mmoles) de chlorure de triéthylsilyle. On agite pendant 28 heures à 20 ° C. On ajoute de l'acétate d'éthyle puis de l'eau. Après décantation, la phase aqueuse est extraite avec de l'acétate d'éthyle. Les phases organiques réunies sont lavées par une solution aqueuse saturée de sulfate de cuivre jusqu'à élimination totale de la pyridine, puis à l'eau et enfin avec une solution aqueuse saturée de chlorure de sodium. Les phases organiques sont séchées sur sulfate de sodium anhydre. Après filtration et évaporation des solvants sous pression réduite, on obtient un résidu (1,35 g) qui est purifié par filtration sur une colonne de gel de silice en éluant avec un mélange dichlorométhane-méthanol (99-1 en volumes). On obtient ainsi, avec un rendement de 83 %, 248 mg (0,354 mmole) de triéthylsilyl-7 baccatine III sous forme d'un solide blanc fondant à 253-254 ° C après recristallisation dans un mélange dichlorométhane-pentane.

## Revendications

**1.** Procédé de préparation du taxol de formule :

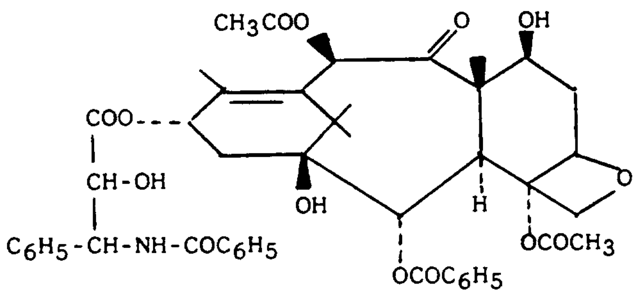

caractérisé en ce que l'on estérifie un dérivé de la phényl-3 isosérine (2R,3S) de formule générale :

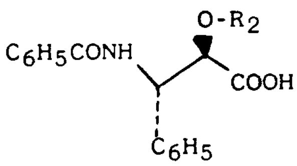

(III)

dans laquelle $R_2$ représente un groupement protecteur de la fonction hydroxy choisi parmi les radicaux méthoxyméthyle, éthoxy-1 éthyle, benzyloxyméthyle, ($\beta$-triméthylsilyléthoxy) méthyle, tétrahydropyrannyle, trichloro-2,2,2 éthoxycarbonyle, par un dérivé du taxane de formule générale :

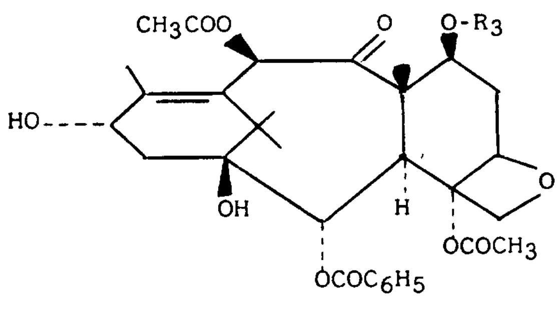

(IV)

dans laquelle $R_3$ représente un groupement protecteur de la fonction hydroxy choisi parmi les radicaux trialkylsilyle dont chaque partie alkyle contient 1 à 3 atomes de carbone, pour obtenir l'ester de formule générale :

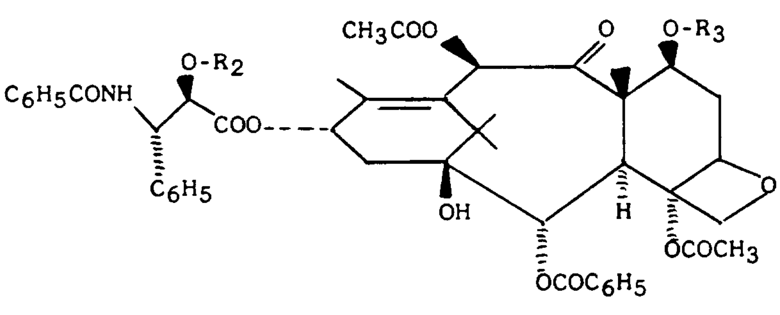

(V)

dans laquelle $R_2$ et $R_3$ sont définis comme précédemment dont on remplace les groupements protecteurs $R_2$ et $R_3$ par des atomes d'hydrogène au moyen d'un acide minéral en solution dans un

alcool aliphatique contenant 1 à 3 atomes de carbone en opérant à une température voisine de 0 °C, puis isole le taxol.

2. Procédé selon la revendication 1 pour la préparation de l'ester de formule générale (IV) telle que défini dans la revendication 1 caractérisé en ce que l'on opère en présence d'un agent de condensation choisi parmi les carbodiimides et les carbonates réactifs et d'un agent d'activation choisi parmi les dialkylaminopyridines, dans un solvant organique aromatique choisi parmi le benzène, le toluène, les xylènes, l'éthylbenzène, l'isopropylbenzène et le chlorobenzène à une température comprise entre 60 et 90 °C.

3. Procédé de préparation selon la revendication 1 caractérisé en ce que le remplacement par des atomes d'hydrogène des groupements protecteurs du produit de formule générale (IV) telle que défini dans la revendication 1 est effectué au moyen d'un acide minéral en solution dans un alcool aliphatique contenant 1 à 3 atomes de carbone en opérant à une température voisine de 0 °C.

4. L'ester de formule générale :

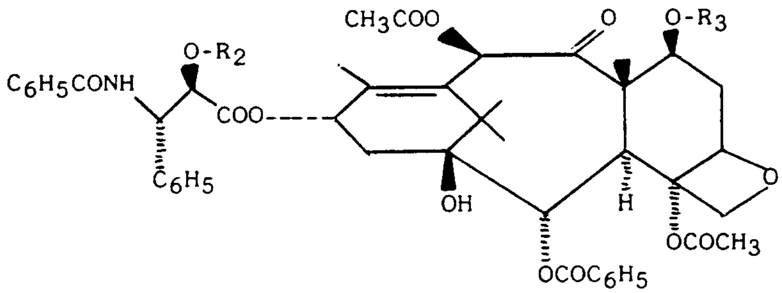

dans laquelle $R_2$ représente un groupement protecteur de la fonction hydroxy choisi parmi les radicaux méthoxyméthyle, éthoxy-1 éthyle, benzyloxyméthyle, ($\beta$-triméthylsilyloxy)méthyle et trichloro-2,2,2 éthoxycarbonyle et $R_3$ représente un groupement protecteur de la fonction hydroxy choisi parmi les radicaux trialkylsilyle dont chaque partie alkyle contient 1 à 3 atomes de carbone.

5. Le dérivé du taxane de formule générale :

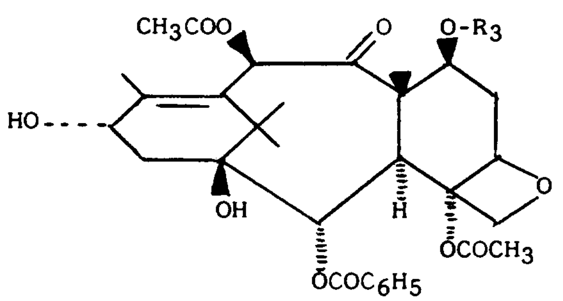

(IV)

dans laquelle $R_3$ représente un groupement protecteur de la fonction hydroxy choisi parmi les radicaux trialkylsilyle dont chaque partie alkyle contient 1 à 3 atomes de carbone.

6. Procédé de préparation d'un dérivé du taxane selon la revendication 5 caractérisé en ce que l'on fait réagir un halogénotrialkylsilane sur la baccatine III ou sur la désacétyl-10 baccatine III suivie dans ce dernier cas de l'acétylation de la trialkylsilyl-7 désacétyl-10 baccatine III intermédiairement obtenue.

7. Procédé selon la revendication 6 caractérisé en ce que l'on fait réagir l'halogénotrialkylsilane à une température voisine de 20 °C en opérant dans un solvant organique basique tel que la pyridine ou dans un solvant organique inerte tel que le chloroforme ou le dichloroéthane en présence d'une amine

tertiaire.

**8.** Procédé selon la revendication 6 caractérisé en ce que l'acétylation de la trialkylsilyl-7 désacétyl-10 baccatine III est effectuée au moyen de chlorure d'acétyle en opérant à une température voisine de 0 ° C en opérant dans un solvant organique basique tel que la pyridine ou dans un solvant organique inerte tel que le chlorure de méthylène, le chloroforme ou le dichloroéthane en présence d'une amine tertiaire.

## Claims

**1.** Process for the preparation of taxol of formula:

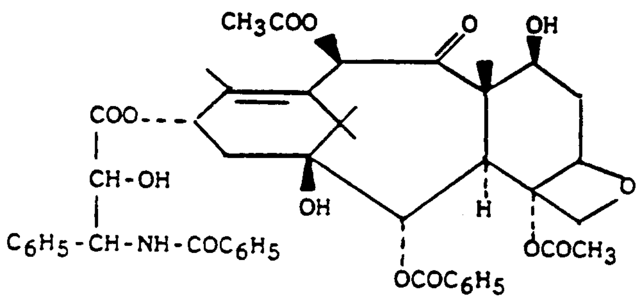

characterised in that a (2R,3S)-3-phenylisoserine derivative of general formula:

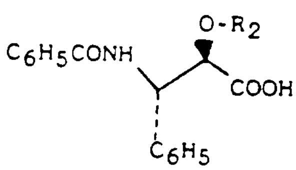

(III)

in which $R_2$ represents a protective group of the hydroxyl functional group chosen from the methoxethyl, 1-ethoxyethyl, benzyloxymethyl, ($\beta$-trimethylsilylethoxy)methyl, tetrahydropyranyl or 2,2,2-trichloroethoxycarbonyl radicals, is esterified with a taxane derivative of general formula:

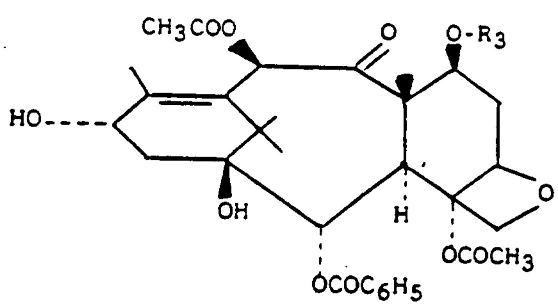

(IV)

in which $R_3$ represents a protective group of the hydroxyl functional group chosen from the trialkylsilyl radicals, each alkyl part of which contains 1 to 3 carbon atoms, to obtain the ester of general formula:

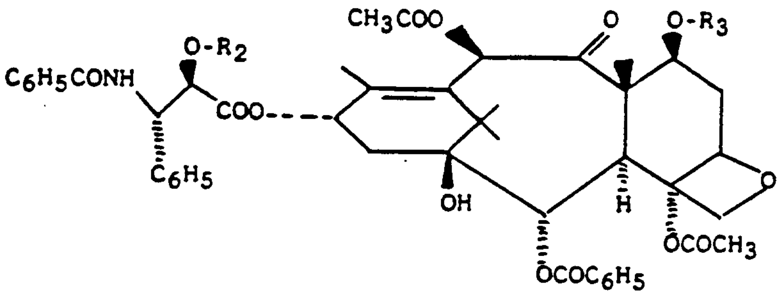

(V)

in which $R_2$ and $R_3$ are defined as above, the protective groups $R_2$ and $R_3$ of which are replaced by hydrogen atoms by means of an inorganic acid in solution in an aliphatic alcohol containing 1 to 3 carbon atoms, the reaction being carried out at a temperature in the region of 0 ° C, and then taxol is isolated.

**2.** Process according to Claim 1 for the preparation of the ester of general formula (V) as defined in Claim 1, characterised in that the preparation is carried out in the presence of a condensing agent chosen from the carbodiimides and the reactive carbonates and of an activating agent chosen from the dialkylaminopyridines, in an aromatic organic solvent chosen from benzene, toluene, xylenes, ethylbenzene, isopropylbenzene and chlorobenzene at a temperature of between 60 and 90 ° C.

**3.** Process of preparation according to Claim 1, characterised in that the replacement of the protective groups of the product of general formula (V) as defined in Claim 1 by hydrogen atoms is carried out by means of an inorganic acid in solution in an aliphatic alcohol containing 1 to 3 carbon atoms, the reaction being carried out at a temperature in the region of 0 ° C.

**4.** The ester of general formula:

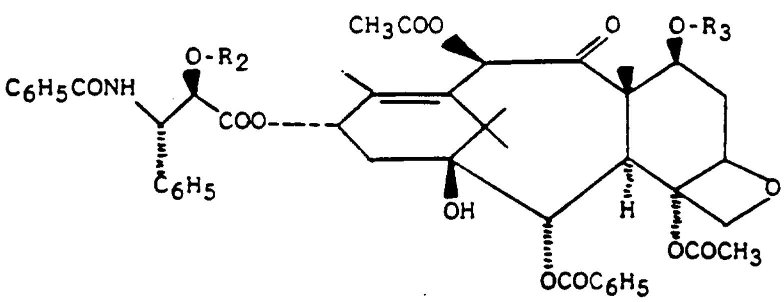

in which $R_2$ represents a protective group of the hydroxyl functional group chosen from the methoxymethyl, 1-ethoxyethyl, benzyloxymethyl, ($\beta$-trimethylsilyloxy)methyl and 2,2,2-trichloroethoxycarbonyl radicals and $R_3$ represents a protective group of the hydroxyl functional group chosen from the trialkylsilyl radicals, each alkyl part of which contains 1 to 3 carbon atoms.

**5.** The taxane derivative of general formula:

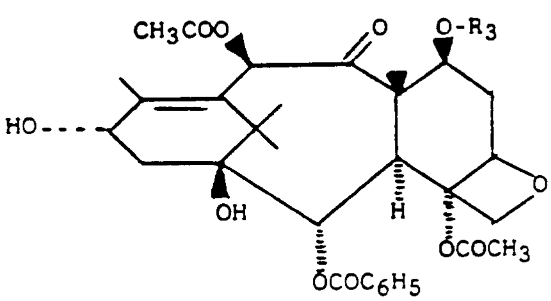

(IV)

in which $R_3$ represents a protective group of the hydroxyl functional group chosen from the trialkylsilyl radicals, each alkyl part of which contains 1 to 3 carbon atoms.

6. Process for the preparation of a taxane derivative according to Claim 5, characterised in that a halotrialkylsilane is reacted with baccatin III or with 10-deacetylbaccatin III, followed, in the latter case, by acetylation of the 7-trialkylsilyl-10-deacetylbaccatin III obtained as an intermediate.

7. Process according to Claim 6, characterised in that the halotrialkylsilane is reacted at a temperature in the region of 20 °C, the reaction being carried out in a basic organic solvent such as pyridine or in an inert organic solvent such as chloroform or dichloroethane in the presence of a tertiary amine.

8. Process according to Claim 6, characterised in that acetylation of 7-trialkylsilyl-10-deacetylbaccatin III is carried out by means of acetyl chloride, the reaction being carried out at a temperature in the region of 0 °C in a basic organic solvent such as pyridine or in an inert organic solvent such as methylene chloride, chloroform or dichloroethane in the presence of a tertiary amine.

## Patentansprüche

1. Verfahren zur Herstellung von Taxol der Formel

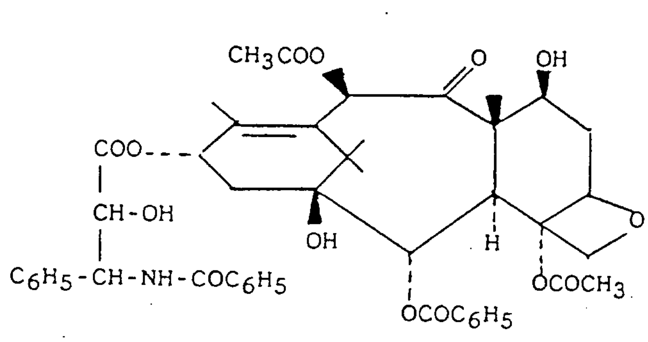

dadurch gekennzeichnet, daß man ein (2R,3S)-3-Phenylisoserinderivat der allgemeinen Formel

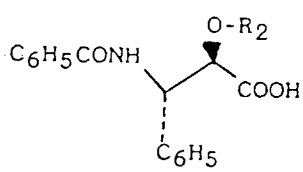

(III)

worin $R_2$ eine Schutzgruppe für die Hydroxyfunktion, ausgewählt unter den Methoxymethyl-, 1-Ethoxyethyl-, Benzyloxymethyl-, ($\beta$-Trimethylsilylethoxy)-methyl-, Tetrahydropyranyl-, 2,2,2-Trichlorethoxycarbonylresten, bedeutet, mit einem Taxanderivat der allgemeinen Formel

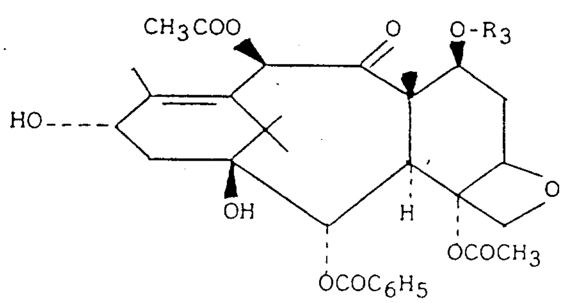

(IV)

worin $R_3$ eine Schutzgruppe für die Hydroxyfunktion, ausgewählt unter den Trialkylsilylresten, von denen jeder Alkylteil 1 bis 3 Kohlenstoffatome enthält, umsetzt, um zu dem Ester der allgemeinen Formel

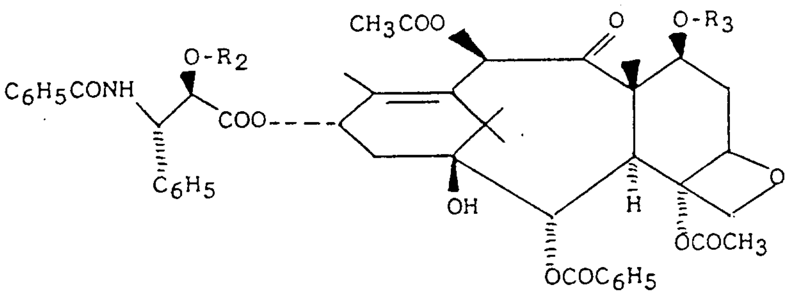

(V)

worin $R_2$ und $R_3$ wie vorstehend definiert sind, zu gelangen, dessen Schutzgruppen $R_2$ und $R_3$ man mit Wasserstoffatomen mittels einer Mineralsäure in Lösung in einem 1 bis 3 Kohlenstoffatome enthaltenden aliphatischen Alkohol ersetzt, wobei man bei einer Temperatur um 0 ° C arbeitet, wonach man das Taxol isoliert.

**2.** Verfahren gemäß Anspruch 1 zur Herstellung des Esters der allgemeinen Formel (IV), wie in Anspruch 1 definiert, dadurch gekennzeichnet, daß man in Gegenwart eines Kondensationsmittels, ausgewählt unter den Carbodiimiden und den reaktiven Carbonaten, und eines Aktivierungsmittels, ausgewählt unter den Dialkylaminopyridinen, in einem aromatischen organischen Lösungsmittel, ausgewählt unter Benzol, Toluol, den Xylolen, Ethylbenzol, Isopropylbenzol und Chlorbenzol, bei einer Temperatur zwischen 60 und 90 ° C arbeitet.

**3.** Verfahren gemäß Anspruch 1, dadurch gekennzeichnet, daß der Austausch durch Wasserstoffatome der Schutzgruppen des Produkts der allgemeinen Formel (IV), wie in Anspruch 1 definiert, mit Hilfe einer Mineralsäure in Lösung in einem 1 bis 3 Kohlenstoffatome aufweisenden aliphatischen Alkohol erfolgt, wobei man bei einer Temperatur um 0 ° C arbeitet.

**4.** Ester der allgemeinen Formel

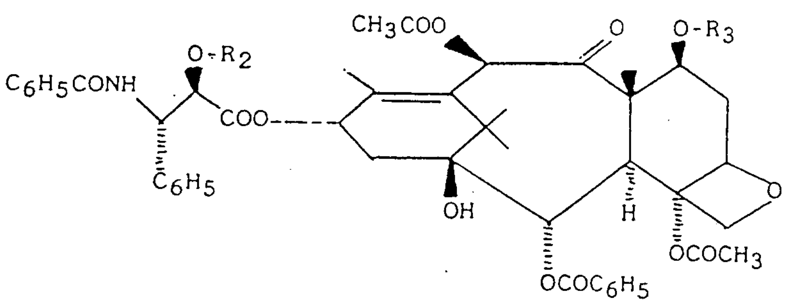

worin $R_2$ eine Schutzgruppe für die Hydroxyfunktion, ausgewählt unter den Methoxymethyl-, 1-Ethoxyethyl-, Benzyloxymethyl-, ($\beta$-Trimethylsilyloxy)-methyl- und 2,2,2-Trichlorethoxycarbonylresten, und $R_3$ eine Schutzgruppe für die Hydroxyfunktion, ausgewählt unter den Trialkylsilylresten, von denen jeder Alkylteil 1 bis 3 Kohlenstoffatome aufweist, bedeuten.

5. Taxanderivat der allgemeinen Formel

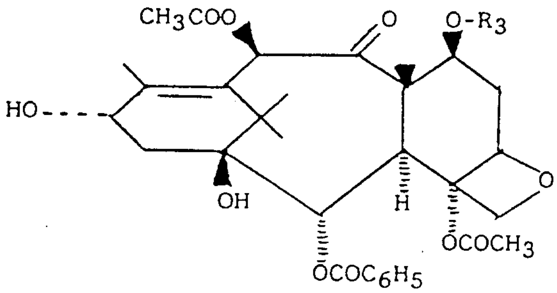

(IV)

worin $R_3$ eine Schutzgruppe für die Hydroxyfunktion, ausgewählt unter den Trialkylsilylresten, von denen jeder Alkylteil 1 bis 3 Kohlenstoffatome aufweist, bedeutet.

6. Verfahren zur Herstellung eines Taxanderivates gemäß Anspruch 5, dadurch gekennzeichnet, daß man ein Halogentrialkylsilan mit Baccatin-III oder mit 10-Desacetylbaccatin-III umsetzt, wonach sich im letztgenannten Fall die Acetylierung des als Zwischenprodukt erhaltenen 7-Trialkylsilyl-10-desacetylbaccatin-III anschließt.

7. Verfahren gemäß Anspruch 6, dadurch gekennzeichnet, daß man Halogentrialkylsilan bei einer Temperatur um 20 °C unter Arbeiten in einem basischen organischen Lösungsmittel, wie Pyridin, oder in einem inerten organischen Lösungsmittel, wie Chloroform oder Dichlorethan, in Gegenwart eines tertiären Amins umsetzt.

8. Verfahren gemäß Anspruch 6, dadurch gekennzeichnet, daß die Acetylierung von 7-Trialkylsilyl-10-desacetylbaccatin-III mit Hilfe von Acetylchlorid unter Arbeiten bei einer Temperatur um 0 °C durchgeführt wird, wobei man in einem basischen organischen Lösungsmittel, wie Pyridin, oder in einem inerten organischen Lösungsmittel, wie Methylenchlorid, Chloroform oder Dichlorethan, in Anwesenheit eines tertiären Amins arbeitet.